# EUROPEAN PATENT SPECIFICATION

(11) **EP 4 398 804 B1**
(45) Date of publication and mention of the grant of the patent: **03.06.2026**
(21) Application number: 22773479.5
(22) Date of filing: 05.09.2022
(51) Int. Cl.: B06B 1/02, B06B 1/06, G01H 3/00, A61B 8/08, A61B 8/12, A61B 8/00

(54) **INTRALUMINAL ULTRASOUND IMAGING ASSEMBLY WITH ELECTRICAL CONNECTION FOR MULTI-ROW TRANSDUCER ARRAY**
INTRALUMINALE ULTRASCHALLBILDGEBUNGSANORDNUNG MIT ELEKTRISCHER VERBINDUNG FÜR MEHRREIHIGE WANDLERANORDNUNG
ENSEMBLE D'IMAGERIE ULTRASONORE INTRALUMINAL AVEC CONNEXION ÉLECTRIQUE POUR RÉSEAU DE TRANSDUCTEURS À RANGÉES MULTIPLES

(30) Priority: 09.09.2021 US 202163242209 P
(43) Date of publication of application: 17.07.2024
(73) Proprietor: Koninklijke Philips N.V., 5656 AE Eindhoven (NL); Philips Image Guided Therapy Corporation, San Diego CA 92130 (US)
(72) Inventor: WILLIAMS, Nathan Andrew, 5656 AG Eindhoven (NL)
(74) Representative: Philips Intellectual Property & Standards
(86) International application number: PCT/EP2022/074631
(87) International publication number: WO 2023/036742

(56) References cited:
- US-A1- 2015 158 052
- US-A1- 2020 101 492
- US-A1- 2020 330 072

## Description

### TECHNICAL FIELD

The present disclosure relates generally to intraluminal ultrasound imaging and, in particular, to the structure of an ultrasound imaging assembly at a distal portion of a catheter or guidewire. For example, a flexible substrate of an ultrasound imaging assembly includes a 2D matrix transducer array that forms a 3D array after the substrate has been rolled into a cylindrical shape.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess a treatment's effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Solid-state (also known as synthetic-aperture) IVUS catheters are one of the two types of IVUS devices commonly used today, the other type being the rotational IVUS catheter. Solid-state IVUS catheters carry a scanner assembly that includes an array of ultrasound transducers distributed around its circumference along with one or more integrated circuit controller chips mounted adjacent to the transducer array. The controllers select individual acoustic elements (or groups of elements) for transmitting an ultrasound pulse and for receiving the ultrasound echo signal. By stepping through a sequence of transmit-receive pairs, the solid-state IVUS system can synthesize the effect of a mechanically scanned ultrasound transducer but without moving parts (hence the solid-state designation). Since there is no rotating mechanical element, the transducer array can be placed in direct contact with the blood and vessel tissue with minimal risk of vessel trauma. Furthermore, because there is no rotating element, the electrical interface is simplified. The solid-state scanner can be wired directly to the imaging system with a simple electrical cable and a standard detachable electrical connector, rather than the complex rotating electrical interface required for a rotational IVUS device.

Current generation IVUS devices include transducer element(s) on a single plane that capture a cross-sectional view of a vessel. Rotational IVUS has one element that rotates while digital phased array IVUS has many stationary elements located around the perimeter of the scanner. Digital phased array IVUS has many benefits including "plug and play" ease of use with a variety of different equipment. However, a single row or plane of transducers cannot readily be used to create 3D images, or to capture movement in three dimensions. Accordingly, a need exists for IVUS devices with more than one row or plane of transducers, and for fabrication methods by which such devices may be readily produced.

### SUMMARY

An intraluminal imaging device, such as an intravascular ultrasound (IVUS) imaging catheter, is described herein. The ultrasound imaging assembly at the distal portion of the catheter includes a flexible substrate rolled into a substantially cylindrical form. The flexible substrate has a distal portion with acoustic elements positioned thereon. The present disclosure provides an array of transducing elements around the perimeter of a cylindrical scanner. This array includes multiple rows of transducing elements to form a grid or matrix. Creating multiple rows of transducing elements in a small form factor, suitable for IVUS applications, can be challenging and expensive. This is particularly challenging because it may be desirable to maintain precise and/or minimal space between each transducer. This grid or matrix of elements is accomplished by mounting a large block of piezoelectric material onto a flex substrate. The flex substrate will include traces that correspond to the location of each transducer or transducing element in the grid/matrix. The large block of piezoelectric material is then diced to create the individual transducing elements. The present disclosure describes a design and assembly process to create such a scanner. The rolled digital transducer matrix disclosed herein has particular, but not exclusive, utility for intraluminal medical devices such as catheters and guidewires.

One general aspect includes an intraluminal imaging device. The intraluminal imaging device includes a flexible elongate member configured to be positioned within a body lumen of a patient, where the flexible elongate member includes a longitudinal axis; an ultrasound imaging assembly disposed at a distal portion of the flexible elongate member, where the ultrasound imaging assembly includes: a flexible substrate including a plurality of conductive traces, where the flexible substrate is disposed around a circumference of the flexible elongate member; and a transducer array disposed on the flexible substrate such that the transducer array is disposed around the circumference of the flexible elongate member, where the transducer array includes a plurality of rows and a plurality of columns, where each column includes a plurality of transducer elements disposed along the longitudinal axis such that the plurality of transducer elements corresponds to the plurality of rows, where the plurality of transducer elements includes a first surface proximate to the flexible substrate and a top surface spaced from the flexible substrate, and where the plurality of transducer elements are electrically coupled to the plurality of conductive traces only on the first surface.

Implementations may include one or more of the following features. In some embodiments, a quantity of the plurality of transducer elements is n and a quantity of the plurality of conductive traces is n+1. In some embodiments, the plurality of conductive traces includes multiple conductive traces electrically coupled to the plurality of transducer elements, respectively, and configured to provide communication of electrical signals associated with ultrasound imaging. In some embodiments, the first surface includes a bottom surface and the second surface includes a top surface, and wherein the bottom surface includes a bottom electrode electrically coupled to a respective conductive trace of the multiple conductive traces. In some embodiments, the plurality of conductive traces includes a single conductive trace at electrical ground and electrically coupled to each transducer element of plurality of transducer elements. In some embodiments, the first surface includes bottom surface and the second surface includes a top surface, and wherein the top surface comprises a top electrode electrically coupled to the single conductive trace. In some embodiments, the plurality of transducer elements includes: a side surface between the top surface and the first surface, where the side surface includes a conductive material electrically coupled to the top electrode and the single conductive trace. In some embodiments, the ultrasound imaging assembly further includes a single electrical connection for the plurality of transducer elements providing the electrical ground via the single conductive trace. In some embodiments, the first surface includes: a first bottom electrode portion in electrical communication with the side surface, the top electrode, and the single conductive trace; and a second bottom electrode portion electrically isolated from the first bottom electrode portion by a discontinuity. In some embodiments, a first transducer element of the plurality of transducer elements and a second transducer element of the plurality of transducer elements are adjacent to one another, where the conductive material on the side surface of the first transducer element is proximate to the conductive material on the side surface of the second transducer element. In some embodiments, the device further including a single electrical connection electrically coupled to the conductive material on the side surface of the first transducer element and to the conductive material on the side surface of the second transducer element. In some embodiments, the transducer array includes gaps between the plurality of transducer elements. In some embodiments, the ultrasound imaging assembly further includes material disposed between side surfaces of the plurality of transducer elements such that no gaps are between the plurality of transducer elements. In some embodiments, the plurality of conductive traces includes a single conductive trace at electrical ground, and where the material is conductive and electrically coupled to the single conductive trace such that the material provides the electrical ground to the plurality of transducer elements. In some embodiments, the top surface includes a top electrode that is continuous across the plurality of transducer elements. The ultrasound imaging assembly further includes a plurality of solder bumps electrically coupling the plurality of transducer elements and the plurality of conductive traces. In some embodiments, the ultrasound imaging assembly further includes an anisotropic conductive material electrically coupling the plurality of transducer elements and the plurality of conductive traces. In some embodiments, the ultrasound imaging assembly further includes a conductive material layer electrically coupling the plurality of transducer elements and the plurality of conductive traces. In some embodiments, the plurality of conductive traces includes a single conductive trace at electrical ground, and where the conductive layer is electrically coupled to the single conductive trace such that the conductive layer provides the electrical ground to the plurality of transducer elements. Implementations of the described techniques may include hardware, a method or process, or computer software on a computer-accessible medium.

One general aspect includes an intravascular ultrasound (IVUS) imaging device which includes a catheter configured to be positioned within a blood vessel of a patient, where the catheter includes a longitudinal axis; an IVUS imaging assembly disposed at a distal portion of the catheter, where the IVUS imaging assembly includes: a flexible substrate including a plurality of conductive traces, where the flexible substrate is disposed around a circumference of the catheter; and a transducer array disposed on the flexible substrate such that the transducer array is disposed around the circumference of the catheter, where the transducer array includes a plurality of rows and a plurality of columns, where each column includes a plurality of transducer elements disposed along the longitudinal axis such that the plurality of transducer elements corresponds to the plurality of rows, where the plurality of transducer elements includes a bottom surface proximate to the flexible substrate and a top surface spaced from the flexible substrate, and where the plurality of transducer elements are electrically coupled to the plurality of conductive traces only on the bottom surface. Other embodiments of this aspect include corresponding computer systems, apparatus, and computer programs recorded on one or more computer storage devices, each configured to perform the actions of the methods.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
**Figure 1** is a schematic diagram of an intraluminal imaging system, according to aspects of the present disclosure.
**Figure 2** is a diagrammatic top view of an ultrasound imaging assembly in a flat configuration, according to aspects of the present disclosure.
**Figure 3** is a diagrammatic perspective view of the ultrasound imaging assembly shown in Fig. 2 in a rolled configuration around a support member, according to aspects of the present disclosure
**Figure 4** is a diagrammatic cross-sectional side view of the ultrasound imaging assembly shown in Fig. 3, according to aspects of the present disclosure.
**Figure 5** is an elevation view of an ultrasound imaging assembly with a distal portion of a flexible substrate in a rolled configuration around a support member, according to aspects of the present disclosure.
**Figure 6** is a perspective view of at least a portion of an example intraluminal imaging device, in accordance with at least one embodiment of the present disclosure.
**Figure 7** is a side view of a piezoelectric block assembly, in accordance with at least one embodiment of the present disclosure.
**Figure 8A** is a side cross-sectional schematic view of at least a portion of an example flexible substrate which includes a plurality of conductive traces, in accordance with at least one embodiment of the present disclosure.
**Figure 8B** is a side cross-sectional schematic view of at least a portion of an example flexible circuit assembly, which includes a flexible substrate that incorporates a plurality of conductive traces, each terminating in a stud bump or solder connection, in accordance with at least one embodiment of the present disclosure.
**Figure 9A** is a side cross-sectional schematic view of an example piezoelectric block assembly mated to an example flexible circuit assembly, in accordance with at least one embodiment of the present disclosure.
**Figure 9B** is a side cross-sectional schematic view of the assembly of Figure 9A, wherein the piezoelectric blocks have been diced into individual transducers, in accordance with at least one embodiment of the present disclosure.
**Figure 10** is a schematic perspective view of at least a portion of an example transducer assembly 1000, in accordance with at least one embodiment of the present disclosure.
**Figure 11** shows another possible configuration of the piezoelectric block 700, in accordance with at least one embodiment of the present disclosure.
**Figure 12** shows a side, cross-sectional schematic view of another possible embodiment of the present disclosure.
**Figure 13** shows a side, cross-sectional schematic view of another possible embodiment of the present disclosure.
**Figures 14A** is a side, cross-sectional perspective view of another embodiment of the present disclosure that incorporates a conductive layer for electrical connection to the transducers.
**Figure 14B** is a side, cross-sectional perspective view of the embodiment of Figure 14A at a later stage of processing
**Figure 14C** is a side, cross-sectional perspective view of the embodiment of Figure 14B at a later stage of processing.
**Figure 15A** is a side, cross-sectional perspective view of another embodiment of the present disclosure wherein the piezoelectric block does not include conductor-filled kerfs or channels.
**Figure 15B** is a side, cross-sectional perspective view of the embodiment of Figure 15A at a later stage of processing.
**Figure 15C** is a side, cross-sectional perspective view of the embodiment of Figure 15B at a later stage of processing.
**Figure 15D** is a side, cross-sectional perspective view of the embodiment of Figure 15C at a later stage of processing.
**Figure 16** is a side, cross-sectional perspective view of another embodiment of the present disclosure wherein trenches or kerfs lned with conductor form a continuous top electrode that wraps around a side of the piezoelectric block to connect with a ground stud bump or solder bump 810-G.
**Figure 17** is a side, cross-sectional perspective view of another embodiment of the present disclosure wherein a piezoelectric block, diced with trenches or kerfs, is filled and covered with a conductive material (e.g., a conductive epoxy, paste, solder, or potting compound), such that the conductive material electrically connects the top ground electrodes 720 to the ground trace.
**Figure 18** is a side, cross-sectional perspective view of another embodiment of the present disclosure wherein the top electrode 720 comprises a conductive film or ribbon (e.g., gold foil or another thin, flexible conductive material) placed (e.g., adhered) across the tops of multiple transducers 212 and down a side of at least one transducer 212 to connect with a ground trace 216-G.
**Figure 19** is a schematic diagram of a processor circuit, in accordance with aspects of the present disclosure.

### DETAILED DESCRIPTION

An intraluminal imaging device, such as an intravascular ultrasound (IVUS) imaging catheter, is described herein. The ultrasound imaging assembly at the distal portion of the catheter includes a flexible substrate. The flexible substrate has a distal portion with acoustic elements positioned thereon, as well as a proximal portion including weld pads to which electrical conductors are attached.

However, existing methods for the construction of such devices make adding additional rows of transducers difficult. In particular, it may be important that these elements are all densely packed into a small space for deliverability (small scanner size) and acoustic purposes. The present disclosure describes the design and accompanying assembly process to create a digital IVUS device with multiple rows of individually addressable piezoelectric elements. This has potential benefits for acoustics, image quality and clinical usage.

Disclosed herein is an array of transducing elements around the perimeter of a cylindrical scanner. This scanner includes multiple rows of transducing elements to form a grid or matrix (e.g., 64 columns and 4 rows of individual transducing elements around the outside perimeter of the device). This concept can be scaled to any number of columns or rows.

Creating multiple rows of transducing elements in a small, 3D form factor suitable for use in IVUS devices can be challenging and expensive. This is particularly challenging because it may be desirable to maintain precise and/or minimal space between each transducer. With the teachings of the present disclosure, this grid or matrix of elements can fabricated by mounting a large block of piezoelectric material onto a flex substrate. The flex substrate can include electrical traces that correspond to the location of each transducer or transducing element in the grid/matrix. The large block of piezoelectric material is then diced to create the individual transducing elements.

The present disclosure describes different designs for such scanners, as well as different fabrication and assembly processes to create them. Having multiple rows of transducing elements has many possible advantages, including: (1) capturing 3 dimensional information without having to move the device. (2) potential to increase pullback speed clinical data integrity. (3) the direction of acoustic energy and the size of the aperture are not limited to one plane. It may therefore be possible to focus acoustic energy or steer it (e.g., with a beamformer) or capture off-plane information (e.g., flow).

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

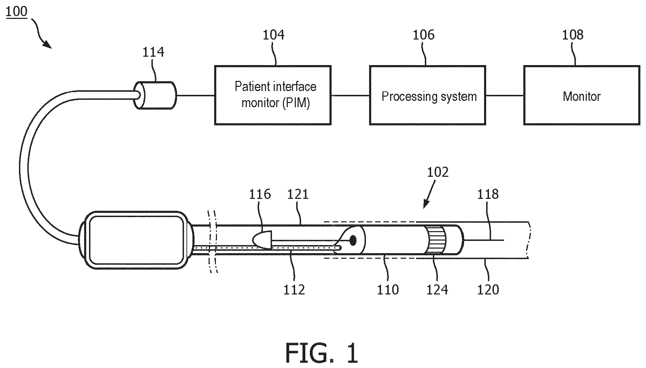

**Figure 1** is a schematic diagram of an intraluminal imaging system 100, according to aspects of the present disclosure. The intraluminal imaging system 100 can be an ultrasound imaging system. In some instances, the system 100 can be an intravascular ultrasound (IVUS) imaging system. The system 100 may include an intraluminal imaging device 102 such as a catheter, guide wire, or guide catheter, a patient interface module (PIM) 104, a processing system or console 106, and a monitor 108. The intraluminal imaging device 102 can be an ultrasound imaging device. In some instances, the device 102 can be an IVUS imaging device, such as a solid-state IVUS device.

At a high level, the IVUS device 102 emits ultrasonic energy from a transducer array 124 included in scanner assembly 110, also referred to as an IVUS imaging assembly, mounted near a distal end of the catheter device. The ultrasonic energy is reflected by tissue structures in the surrounding medium, such as a vessel 120, or another body lumen surrounding the scanner assembly 110, and the ultrasound echo signals are received by the transducer array 124. In that regard, the device 102 can be sized, shaped, or otherwise configured to be positioned within the body lumen of a patient. The PIM 104 transfers the received echo signals to the console or computer 106 where the ultrasound image (including flow information in some embodiments) is reconstructed and displayed on the monitor 108. The console or computer 106 can include a processor and a memory. The computer or computing device 106 can be operable to facilitate the features of the IVUS imaging system 100 described herein. For example, the processor can execute computer readable instructions stored on the non-transitory tangible computer readable medium.

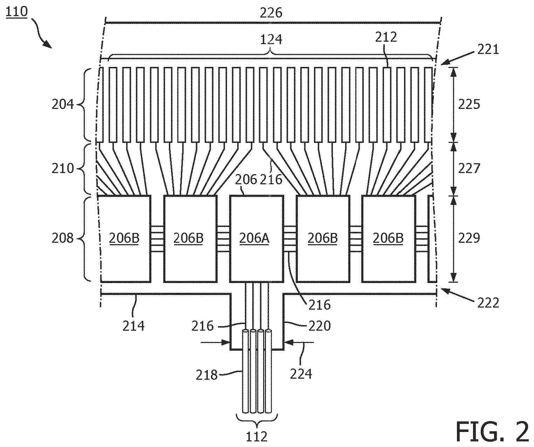

The PIM 104 facilitates communication of signals between the IVUS console 106 and the scanner assembly 110 included in the IVUS device 102. This communication includes the steps of: (1) providing commands to integrated circuit controller chip(s) 206A and 206B, illustrated in Fig. 2, included in the scanner assembly 110 to select the particular transducer array element(s), or acoustic element(s), to be used for transmit and receive, (2) providing the transmit trigger signals to the integrated circuit controller chip(s) 206A and 206B (Fig. 2) included in the scanner assembly 110 to activate the transmitter circuitry to generate an electrical pulse to excite the selected transducer array element(s), and/or (3) accepting amplified echo signals received from the selected transducer array element(s) via amplifiers included on the integrated circuit controller chip(s) 126 of the scanner assembly 110. In some embodiments, the PIM 104 performs preliminary processing of the echo data prior to relaying the data to the console 106. In examples of such embodiments, the PIM 104 performs amplification, filtering, and/or aggregating of the data. In an embodiment, the PIM 104 also supplies high- and low-voltage DC power to support operation of the device 102 including circuitry within the scanner assembly 110.

The IVUS console 106 receives the echo data from the scanner assembly 110 by way of the PIM 104 and processes the data to reconstruct an image of the tissue structures in the medium surrounding the scanner assembly 110. The console 106 outputs image data such that an image of the vessel 120, such as a cross-sectional image of the vessel 120, is displayed on the monitor 108. The vessel 120 may represent fluid filled or surrounded structures, both natural and man-made. The vessel 120 may be within a body of a patient. The vessel 120 may be a blood vessel, such as an artery or a vein of a patient's vascular system, including cardiac vasculature, peripheral vasculature, neural vasculature, renal vasculature, and/or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

In some embodiments, the IVUS device includes some features similar to traditional solid-state IVUS catheters, such as the EagleEye^{®} catheter available from Volcano Corporation and those disclosed in U.S. Patent No. 7,846,101. For example, the IVUS device 102 includes the scanner assembly 110 near a distal end of the device 102 and a transmission line bundle 112 extending along the longitudinal body of the device 102. The transmission line bundle or cable 112 can include a plurality of conductors, including one, two, three, four, five, six, seven, or more conductors 218 (Fig. 2). It is understood that any suitable gauge wire can be used for the conductors 218. In an embodiment, the cable 112 can include a four-conductor transmission line arrangement with, e.g., 41 AWG gauge wires. In an embodiment, the cable 112 can include a seven-conductor transmission line arrangement utilizing, e.g., 44 AWG gauge wires. In some embodiments, 43 AWG gauge wires can be used.

The transmission line bundle 112 terminates in a PIM connector 114 at a proximal end of the device 102. The PIM connector 114 electrically couples the transmission line bundle 112 to the PIM 104 and physically couples the IVUS device 102 to the PIM 104. In an embodiment, the IVUS device 102 further includes a guide wire exit port 116. Accordingly, in some instances the IVUS device is a rapid-exchange catheter. The guide wire exit port 116 allows a guide wire 118 to be inserted towards the distal end in order to direct the device 102 through the vessel 120.

**Figure 2** is a diagrammatic top view of a portion of a flexible assembly 110, according to aspects of the present disclosure. The flexible assembly 110 includes a transducer array 124 formed in a transducer region 204 and transducer control logic dies 206 (including dies 206A and 206B) formed in a control region 208, with a transition region 210 disposed therebetween. The transducer array 124 includes an array of ultrasound transducer elements 212. The transducer control logic dies 206 are mounted on a flexible substrate 214 into which the transducer elements 212 have been previously integrated. The flexible substrate 214 is shown in a flat configuration in Fig. 2. Though six control logic dies 206 are shown in Fig. 2, any number of control logic dies 206 may be used. For example, one, two, three, four, five, six, seven, eight, nine, ten, or more control logic dies 206 may be used.

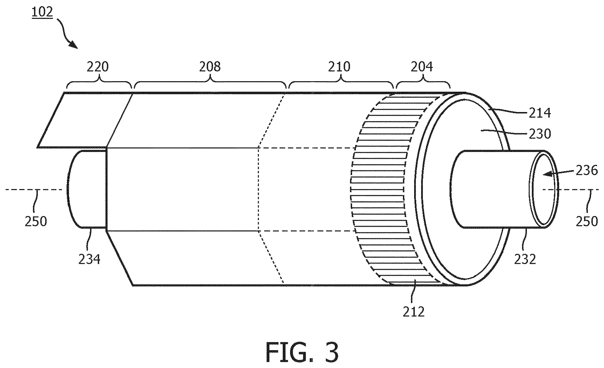

The flexible substrate 214, on which the transducer control logic dies 206 and the transducer elements 212 are mounted, provides structural support and interconnects for electrical coupling. The flexible substrate 214 may be constructed to include a film layer of a flexible polyimide material such as KAPTON^{™} (trademark of DuPont). Other suitable materials include polyester films, polyimide films, polyethylene napthalate films, or polyetherimide films, liquid crystal polymer, other flexible printed semiconductor substrates as well as products such as Upilex^{®} (registered trademark of Ube Industries) and TEFLON^{®} (registered trademark of E.I. du Pont). In the flat configuration illustrated in Fig. 2, the flexible substrate 214 has a generally rectangular shape. As shown and described herein, the flexible substrate 214 is configured to be wrapped around a support member 230 (Fig. 3) in some instances. Therefore, the thickness of the film layer of the flexible substrate 214 is generally related to the degree of curvature in the final assembled flexible assembly 110. In some embodiments, the film layer is between 5 µm and 100 µm, with some particular embodiments being between 5 µm and 25.1 µm, e.g., 6 µm.

The set of transducer control logic dies 206 is a non-limiting example of a control circuit. The transducer region 204 is disposed at a distal portion 221 of the flexible substrate 214. The control region 208 is disposed at a proximal portion 222 of the flexible substrate 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or, the length 227 of the transition region 210 may be less than lengths 225 and 229, the length 227 of the transition region 210 can be greater than lengths 225, 229 of the transducer region and controller region, respectively.

The control logic dies 206 are not necessarily homogenous. In some embodiments, a single controller is designated a master control logic die 206A and contains the communication interface for cable 112, between a processing system, e.g., processing system 106, and the flexible assembly 110. Accordingly, the master control circuit may include control logic that decodes control signals received over the cable 112, transmits control responses over the cable 112, amplifies echo signals, and/or transmits the echo signals over the cable 112. The remaining controllers are slave controllers 206B. The slave controllers 206B may include control logic that drives a transducer element 212 to emit an ultrasonic signal and selects a transducer element 212 to receive an echo. In the depicted embodiment, the master controller 206A does not directly control any transducer elements 212. In other embodiments, the master controller 206A drives the same number of transducer elements 212 as the slave controllers 206B or drives a reduced set of transducer elements 212 as compared to the slave controllers 206B. In an exemplary embodiment, a single master controller 206A and eight slave controllers 206B are provided with eight transducers assigned to each slave controller 206B.

To electrically interconnect the control logic dies 206 and the transducer elements 212, in an embodiment, the flexible substrate 214 includes conductive traces 216 formed in the film layer that carry signals between the control logic dies 206 and the transducer elements 212. In particular, the conductive traces 216 providing communication between the control logic dies 206 and the transducer elements 212 extend along the flexible substrate 214 within the transition region 210. In some instances, the conductive traces 216 can also facilitate electrical communication between the master controller 206A and the slave controllers 206B. The conductive traces 216 can also provide a set of conductive pads that contact the conductors 218 of cable 112 when the conductors 218 of the cable 112 are mechanically and electrically coupled to the flexible substrate 214. Suitable materials for the conductive traces 216 include copper, gold, aluminum, silver, tantalum, nickel, and tin, and may be deposited on the flexible substrate 214 by processes such as sputtering, plating, and etching. In an embodiment, the flexible substrate 214 includes a chromium adhesion layer. The width and thickness of the conductive traces 216 are selected to provide proper conductivity and resilience when the flexible substrate 214 is rolled. In that regard, an exemplary range for the thickness of a conductive trace 216 and/or conductive pad is between 1-5 µm. For example, in an embodiment, 5 µm conductive traces 216 are separated by 5 µm of space. The width of a conductive trace 216 on the flexible substrate may be further determined by the width of the conductor 218 to be coupled to the trace or pad.

The flexible substrate 214 can include a conductor interface 220 in some embodiments. The conductor interface 220 can be in a location of the flexible substrate 214 where the conductors 218 of the cable 112 are coupled to the flexible substrate 214. For example, the bare conductors of the cable 112 are electrically coupled to the flexible substrate 214 at the conductor interface 220. The conductor interface 220 can be tab extending from the main body of flexible substrate 214. In that regard, the main body of the flexible substrate 214 can refer collectively to the transducer region 204, controller region 208, and the transition region 210. In the illustrated embodiment, the conductor interface 220 extends from the proximal portion 222 of the flexible substrate 214. In other embodiments, the conductor interface 220 is positioned at other parts of the flexible substrate 214, such as the distal portion 221, or the flexible substrate 214 may lack the conductor interface 220. A value of a dimension of the tab or conductor interface 220, such as a width 224, can be less than the value of a dimension of the main body of the flexible substrate 214, such as a width 226. In some embodiments, the substrate forming the conductor interface 220 is made of the same material(s) and/or is similarly flexible as the flexible substrate 214. In other embodiments, the conductor interface 220 is made of different materials and/or is comparatively more rigid than the flexible substrate 214. For example, the conductor interface 220 can be made of a plastic, thermoplastic, polymer, hard polymer, etc., including polyoxymethylene (e.g., DELRIN^{®}), polyether ether ketone (PEEK), nylon, Liquid Crystal Polymer (LCP), and/or other suitable materials.

**Figure 3** illustrates a perspective view of the scanner assembly 110 in a rolled configuration. In some instances, the flexible substrate 214 is transitioned from a flat configuration (Fig. 2) to a rolled or more cylindrical configuration (Fig. 3). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND SENSING ASSEMBLY HAVING A FLEXIBLE SUBSTRATE."

Depending on the application and embodiment of the presently disclosed invention, transducer elements 212 may be piezoelectric transducers, single crystal transducer, or PZT (lead zirconate titanate) transducers. In other embodiments, the transducer elements of transducer array 124 may be flexural transducers, piezoelectric micromachined ultrasonic transducers (PMUTs), capacitive micromachined ultrasonic transducers (CMUTs), or any other suitable type of transducer element. In such embodiments, transducer elements 212 may comprise an elongate semiconductor material or other suitable material that allows micromachining or similar methods of disposing extremely small elements or circuitry on a substrate.

In some embodiments, the transducer elements 212 and the controllers 206 can be positioned in an annular configuration, such as a circular configuration or in a polygon configuration, around a longitudinal axis 250 of a support member 230. It is understood that the longitudinal axis 250 of the support member 230 may also be referred to as the longitudinal axis of the scanner assembly 110, the flexible elongate member 121, or the device 102. For example, a cross-sectional profile of the imaging assembly 110 at the transducer elements 212 and/or the controllers 206 can be a circle or a polygon. Any suitable annular polygon shape can be implemented, such as one based on the number of controllers or transducers, flexibility of the controllers or transducers, etc. Some examples may include a pentagon, hexagon, heptagon, octagon, nonagon, decagon, etc. In some examples, the transducer controllers 206 may be used for controlling the ultrasound transducers 212 to obtain imaging data associated with the vessel 120.

The support member 230 can be referenced as a unibody in some instances. The support member 230 can be composed of a metallic material, such as stainless steel, or a non-metallic material, such as a plastic or polymer as described in U.S. Provisional Application No. 61/985,220, "Pre-Doped Solid Substrate for Intravascular Devices," filed April 28, 2014, ('220 Application). In some embodiments, support member 230 may be composed of 303 stainless steel. The support member 230 can be a ferrule having a distal flange or portion 232 and a proximal flange or portion 234. The support member 230 can be tubular in shape and define a lumen 236 extending longitudinally therethrough. The lumen 236 can be sized and shaped to receive the guide wire 118. The support member 230 can be manufactured using any suitable process. For example, the support member 230 can be machined and/or electrochemically machined or laser milled, such as by removing material from a blank to shape the support member 230, or molded, such as by an injection molding process or a micro injection molding process.

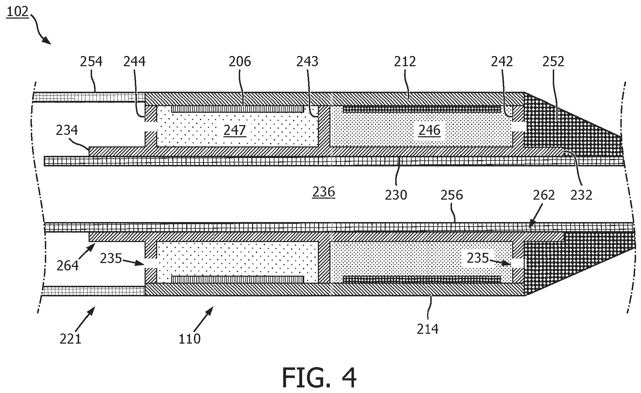

**Figure 4** is a diagrammatic cross-sectional side view of a distal portion of the intraluminal imaging device 102, including the flexible substrate 214 and the support member 230, according to aspects of the present disclosure. The lumen 236 may be connected with the entry/exit port 116 and is sized and shaped to receive the guide wire 118 (Fig. 1). In some embodiments, the support member 230 may be integrally formed as a unitary structure, while in other embodiments the support member 230 may be formed of different components, such as a ferrule and stands 242, 243, and 244, that are fixedly coupled to one another. In some cases, the support member 230 and/or one or more components thereof may be completely integrated with inner member 256. In some cases, the inner member 256 and the support member 230 may be joined as one, e.g., in the case of a polymer support member.

Stands 242, 243, and 244 that extend vertically are provided at the distal, central, and proximal portions respectively, of the support member 230. The stands 242, 243, and 244 elevate and support the distal, central, and proximal portions of the flexible substrate 214. In that regard, portions of the flexible substrate 214, such as the transducer portion 204 (or transducer region 204), can be spaced from a central body portion of the support member 230 extending between the stands 242, 243, and 244. The stands 242, 243, 244 can have the same outer diameter or different outer diameters. For example, the distal stand 242 can have a larger or smaller outer diameter than the central stand 243 and/or proximal stand 244 and can also have special features for rotational alignment as well as control chip placement and connection.

To improve acoustic performance, the cavity between the transducer array 212 and the surface of the support member 230 may be filled with an acoustic backing material 246. The liquid backing material 246 can be introduced between the flexible substrate 214 and the support member 230 via passageway 235 in the stand 242, or through additional recesses as will be discussed in more detail hereafter. The backing material 246 may serve to attenuate ultrasound energy emitted by the transducer array 212 that propagates in the undesired, inward direction.

The cavity between the circuit controller chips 206 and the surface of the support member 230 may be filled with an underfill material 247. The underfill material 247 may be an adhesive material (e.g. an epoxy) which provides structural support for the circuit controller chips 206 and/or the flexible substrate 214. The underfill 247 may additionally be any suitable material.

In some embodiments, the central body portion of the support member can include recesses allowing fluid communication between the lumen of the unibody and the cavities between the flexible substrate 214 and the support member 230. Acoustic backing material 246 and/or underfill material 247 can be introduced via the cavities (during an assembly process, prior to the inner member 256 extending through the lumen of the unibody. In some embodiments, suction can be applied via the passageways 235 of one of the stands 242, 244, or to any other suitable recess while the liquid backing material 246 is fed between the flexible substrate 214 and the support member 230 via the passageways 235 of the other of the stands 242, 244, or any other suitable recess. The backing material can be cured to allow it to solidify and set. In various embodiments, the support member 230 includes more than three stands 242, 243, and 244, only one or two of the stands 242, 243, 244, or none of the stands. In that regard the support member 230 can have an increased diameter distal portion 262 and/or increased diameter proximal portion 264 that is sized and shaped to elevate and support the distal and/or proximal portions of the flexible substrate 214.

The support member 230 can be substantially cylindrical in some embodiments. Other shapes of the support member 230 are also contemplated including geometrical, non-geometrical, symmetrical, non-symmetrical, cross-sectional profiles. As the term is used herein, the shape of the support member 230 may reference a cross-sectional profile of the support member 230. Different portions of the support member 230 can be variously shaped in other embodiments. For example, the proximal portion 264 can have a larger outer diameter than the outer diameters of the distal portion 262 or a central portion extending between the distal and proximal portions 262, 264. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

A proximal inner member 256 and a proximal outer member 254 are coupled to the proximal portion 264 of the support member 230. The proximal inner member 256 and/or the proximal outer member 254 can comprise a flexible elongate member. The proximal inner member 256 can be received within a proximal flange 234. The proximal outer member 254 abuts and is in contact with the proximal end of flexible substrate 214. A distal tip member 252 is coupled to the distal portion 262 of the support member 230. For example, the distal member 252 is positioned around the distal flange 232. The tip member 252 can abut and be in contact with the distal end of flexible substrate 214 and the stand 242. In other embodiments, the proximal end of the tip member 252 may be received within the distal end of the flexible substrate 214 in its rolled configuration. In some embodiments there may be a gap between the flexible substrate 214 and the tip member 252. The distal member 252 can be the distal-most component of the intraluminal imaging device 102. The distal tip member 252 may be a flexible, polymeric component that defines the distal-most end of the imaging device 102. The distal tip member 252 may additionally define a lumen in communication with the lumen 236 defined by support member 230. The guide wire 118 may extend through lumen 236 as well as the lumen defined by the tip member 252.

One or more adhesives can be disposed between various components at the distal portion of the intraluminal imaging device 102. For example, one or more of the flexible substrate 214, the support member 230, the distal member 252, the proximal inner member 256, the transducer array 212, and/or the proximal outer member 254 can be coupled to one another via an adhesive. Stated differently, the adhesive can be in contact with e.g. the transducer array 212, the flexible substrate 214, the support member 230, the distal member 252, the proximal inner member 256, and/or the proximal outer member 254, among other components.

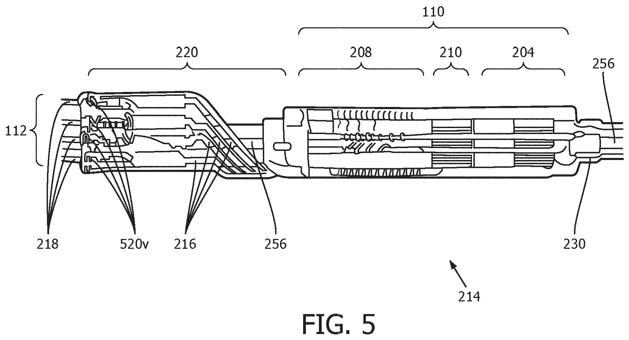

**Figure 5** is perspective view of an ultrasound imaging assembly 110 with a distal portion of a flexible substrate 214 in a rolled configuration around the support member 230, in accordance with aspects of the present disclosure. The flexible scanner assembly 110 has been wrapped around the support member 230 (e.g., a ferrule, metal tube, unibody, or other suitable structure) such that the control region 208, transition region 210, and transducer region 204 have taken a cylindrical shape around the support member 230. In some instances, the control region 208, transition region 210, and transducer region 204 can be referenced as a distal portion or scanner body portion of the flexible substrate 214. Also visible is the conductor interface or attachment portion 220, which includes conductive traces 216 and conductive weld pads or solder pads 520 to which may be attached the conductors 218 that form the cable 112. In some instances, the conductor interface 220 can be referenced as a proximal portion of the flexible substrate 214. The conductive traces 216 in the conductor interface or attachment portion 220 establish electrical communication between the weld pads 520 and the controller region 208. In order to prevent heat damage to the scanner assembly 110 when conductors 218 are welded or soldered to the conductive pads 520, the conductor interface 220 projects away from the control region 208 of the scanner assembly 110 for some distance.

The cable 112 includes a plurality of conductors 218 extending along a length of the flexible elongate member (e.g., flexible elongate member 121 as shown for example in Figure 1). A proximal region of the attachment portion comprises the plurality of electrical contacts or weld pads 520 that are coupled to the plurality of conductive traces 216. The plurality of conductors 218 are electrically connected to the plurality of conductive traces 216 via the plurality of electrical contacts or weld pads or solder pads 520. The cable 112 formed by the plurality of electrical conductors 218 extends within an annular space between the inner member 256 and the outer member (e.g., outer member 254 as shown for example in Figure 4). The proximal region of the attachment portion comprises a generally rectangular shape and is aligned with the longitudinal axis of the flexible elongate member. The scanner body portion or scanner assembly 110 of the device (e.g., device 102 as shown for example in Figure 1) is positioned around the rigid tubular member or support member 230. The attachment portion 220 extends proximally of the rigid tubular member, the one or more transducer elements of the transducer region 204, and the one or more control circuits of the control region 208.

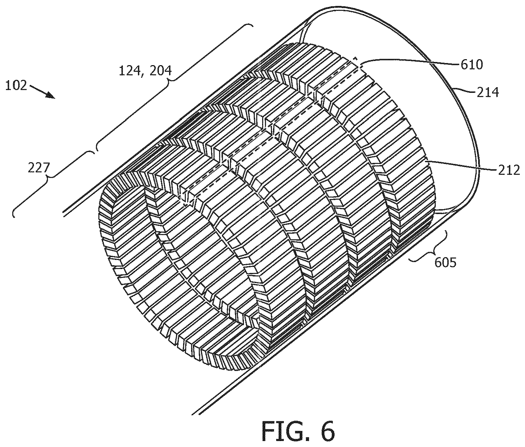

**Figure 6** is a perspective view of at least a portion of an example intraluminal imaging device 102, in accordance with at least one embodiment of the present disclosure. Visible in Figure 6 are the flexible substrate 214 in a rolled configuration, including the transition region 227 and the transducer region 204. The transducer region 204 incudes a cylindrical transducer array 124 composed of individual ultrasound transducers or transducing elements 212. In this example, four rings or rows 605 of transducers are shown, with each ring or row comprising 64 transducers. Other numbers of rows 605 may be used instead or in addition, including but not limited to one row, two rows, three rows, four rows, eight rows, sixteen rows, or more. Other numbers of transducers per row, both larger and smaller, may be used instead or in addition, such as 4, 8, 16, 32, 128, 256, 512, or more transducers per row. Each column 610 of the array 124 can include a quantity of transducer elements equal to the quantity of rows 605. In that regard, each transducer element in the column 610 corresponds to a different row 605 of the array 124. Similarly, each row 605 of the array 124 can include a quantity of transducer elements equal to the quantity of columns 610. In that regard, each transducer element in the row 605 corresponds to a different column 610 of the array 124. The rows 605 can be oriented perpendicular to the columns 610 in the array 124.

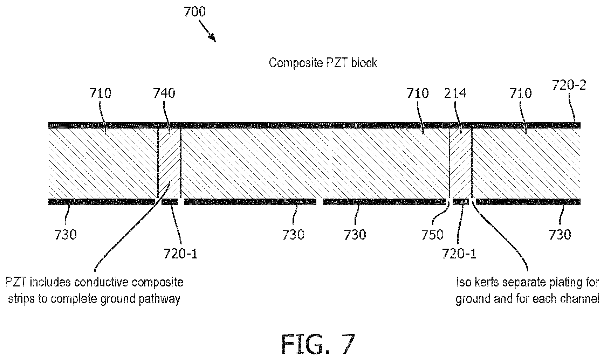

**Figure 7** is a side view of a piezoelectric block assembly 700, in accordance with at least one embodiment of the present disclosure. The piezoelectric block assembly 700 includes a block of piezoelectric material (e.g., a lead zirconate titanate or PZT material, or other piezoelectric material) divided into subunits 710 that are separated by spacers of a conductive filler material 740 (e.g., a conductive composite such as a metallic epoxy). The piezoelectric block assembly 700 includes an electrode coating 720-2 (e.g., a metal or other conductive material) on the top surface that can serve as one or more ground electrodes or negative terminals. In addition, the piezoelectric block assembly 700 includes an electrode coating on the bottom surface, which is divided by discontinuities 750 into separately addressable actuation electrodes or positive terminals 730, and ground electrodes or negative terminals 720-1, which make electrical contact with the top-surface ground electrode 720-2 via the conductive filler material 740. The discontinuities can be isolations kerfs in an exemplary embodiments. Electrode 720-1 can be referenced as one bottom electrode portion and electrode 730 can be referenced as another bottom electrode portion. These electrode portions are electrically isolated from one another.

As will be appreciated by a person of ordinary skill in the art, other arrangements are possible, and fall within the scope of the present disclosure. For example, depending on the implementation, the electrodes 720-1 and 720-2 may be used as positive terminals, while the electrodes 730 may be used as negative terminals. Relative dimensions and thicknesses of materials may be different than what is shown in the Figure. Instead of isolation kerfs 750, other techniques (such as masking, photolithography, etc.) may also be used to create discontinuity inand/or interrupt the bottom metallic coating between elements to divide it into separately addressable electrodes.

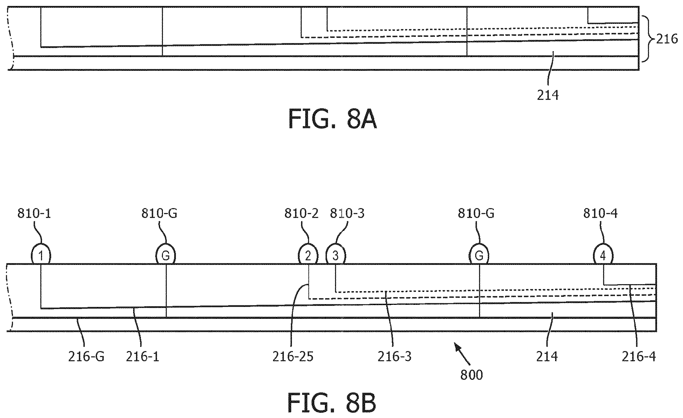

**Figure 8A** is a side cross-sectional schematic view of at least a portion of an example flexible substrate 214, which includes a plurality of conductive traces 216, in accordance with at least one embodiment of the present disclosure. The conductive traces 216 may include mutually interconnected ground traces and/or mutually isolated, individually addressable control traces for carrying power and signals to individual transducers or transducing elements 212 of the transducer array 124 (as shown for example in Figure 2). In some cases, each transducer 212 may be referred to as a channel. In other cases, a channel may comprise multiple transducers. Figure 8A represents an example substrate 214 which includes individual circuits for each of four channels, and a common ground circuit that connects to all elements. Other arrangements may be used instead or in addition to control the individual channels. The conductive traces 216 may be on the surface of the substrate 214, or may exist at one or more layers inside the substrate 214. One or a plurality of the conductive traces 216 can provide electrical communication of electrical signals associated with ultrasound imaging (e.g., transmit trigger signals, electrical signals representative of received ultrasound echoes, etc.). One or a plurality of the conductive traces 216 can provide electrical connection with electrical ground for the transducer elements 212 of the array 124. The illustrated embodiment, five conductive traces 216 are provided: four of the conductive traces 216 provide electrical communication of electrical signals associated with ultrasound imaging and one of the conductive traces 216 provide electrical connection with electrical ground for the transducer elements.

**Figure 8B** is a side cross-sectional schematic view of at least a portion of an example flexible circuit assembly 800, which includes a flexible substrate 214 that incorporates a plurality of conductive traces 216, each terminating in a stud bump or solder connection 810, in accordance with at least one embodiment of the present disclosure. In the example shown in Figure 8B, a Ground conductive trace 216-G connects to two stud bumps or solder connections 810-G, while a Channel 1 conductive trace 216-1 connects to a single stud bump or solder connection 810-1, a Channel 2 conductive trace 216-2 connects to a single stud bump or solder connection 810-2, a Channel 3 conductive trace 216-3 connects to a single stud bump or solder connection 810-3, a Channel 4 conductive trace 216-4 connects to a single stud bump or solder connection 810-4. A person of ordinary skill in the art will appreciate that this exemplary arrangement, or other comparable arrangements that fall within the scope of the present disclosure, can be scaled up to accommodate an arbitrarily large number of channels or individual transducers.

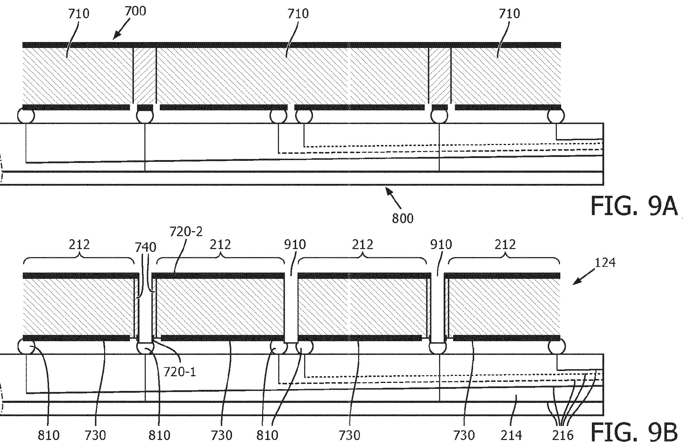

**Figure 9A** is a side cross-sectional schematic view of an example piezoelectric block assembly 700 mated to an example flexible circuit assembly 800, in accordance with at least one embodiment of the present disclosure. The alignment of the piezoelectric block assembly 700 with the flexible circuit assembly 800 is such that each piezoelectric block 710 can be actuated by a solder bump that is addressed by a control circuit trace (as shown above in Figure 8B) and drains to a common ground through a solder bump connected to a ground trace (as shown above in Figure 8B). However, in some cases the presence of conductive filler material between the piezoelectric blocks may interfere with individual actuation of the blocks.

**Figure 9B** is a side cross-sectional schematic view of the assembly of Figure 9A, wherein the piezoelectric blocks have been diced into individual transducers 212, in accordance with at least one embodiment of the present disclosure. Dicing channels 910 have been cut through the piezoelectric blocks 710 and the conductive filler material 740, forming individual transducers 212. The individual transducers 212 can thus have a gap 910 in between. Each transducer 212 has a top ground electrode 720-2 that is electrically connected to one or more bottom ground electrodes 720-1 through the conductive filler material 740, such that it can drain through a ground trace 216 connected to a solder bumps 810. Each transducer 212 also has an actuation electrode 730 that can be energized by a control trace 216 through a stud bump 810. When the solder bumps 810 are addressed individually by the control traces 216 (as shown above in Figure 8B), each ultrasound transducer 212 can be individually actuated to produce an ultrasound pulse and to receive an ultrasound echo. The received echo then produces an electrical signal that travels back through the traces 216. A quantity of the transducer elements 212 can be N and a quantity of the plurality of conductive traces 216 can be N+1. In the illustrated embodiment, the quantity of the transducer elements 212 is four and a quantity of the plurality of conductive traces 216 can be 5. The four transducer elements 212 can be representative of a column 610 of transducer elements in the array 124 (Fig. 6.)

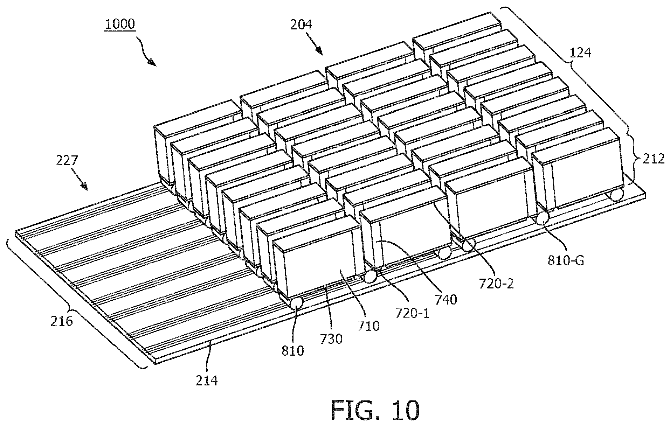

**Figure 10** is a schematic perspective view of at least a portion of an example transducer assembly 1000, in accordance with at least one embodiment of the present disclosure. In the example shown in Figure 10, the transducer region assembly 1000 includes flexible substrate 214 that includes a transition region 227 and a transducer region 204. The transition region 227 includes a plurality of conductive traces 216. The transducer region 204 includes a transducer array 124 which includes a plurality of transducers 212.

In the example shown in Figure 10, each transducer 212 includes a dedicated stud bump or solder bump 810 electrically connected to a bottom electrode 730 for actuating the transducer 212, and a shared stud bump or solder bump 810-G that connects to a common ground. Each transducer 212 also includes a conductive material 740 along one side, which electrically connects the shared stud bump 810-G, via a negative terminal 720-1, to a top electrode 720-2 that serves as a ground for the transducer 212.

Dicing and/or other microchip processing techniques can be used to separate a piezoelectric block 700 (as shown for example in Figure 7) into multiple columns and/or rows of transducing elements or transducers 212. The figure illustrates what the grid or array 124 of transducers 212 may look like in three dimensions. The flexible substrate 214 can then be rolled to create a curve or cylinder as shown for example in Figure 6.

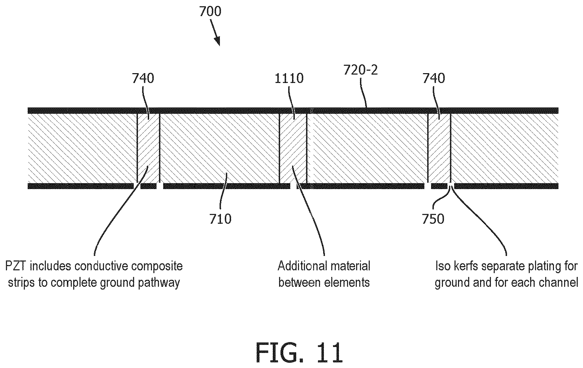

**Figure 11** shows another possible configuration of the piezoelectric block 700, in accordance with at least one embodiment of the present disclosure. Many variants of the transducer array are possible, and can be implemented individually or in a large plurality of combinations, all of which fall within the scope of the present disclosure.

For example, transducers may include any piezoelectric material, including standard PZT, single crystal PZT, or others. The conductive material in the composite piezoelectric block may be any variety of material selected for mechanical, acoustic or manufacturing properties. The device may be formed such that the substrate is on the outer perimeter of the piezoelectric material (making it part of the acoustic window) or on the inner perimeter of the piezoelectric material. It may be desirable to fill the kerfs rather than leaving a gap between elements. If so, this fill may be added after the above-mentioned processing. Alternatively, the piezoelectric block may be designed such that there are composite materials placed between every element. In this way, the piezoelectric block would not need to be diced into separate transducing elements.

In the example shown in Figure 11, the piezoelectric block 700 includes not only channels filled with conductive material 740, but also alternating channels (e.g., in between the conductor-filled channels) that are filled with an insulating material 1110. As one possible advantage, this may permit the top electrode 720-2 to be continuous across all of the transducers. In the illustrated embodiment, material fills the space between transducer elements 710 such that no gap is present between the transducer elements 710.

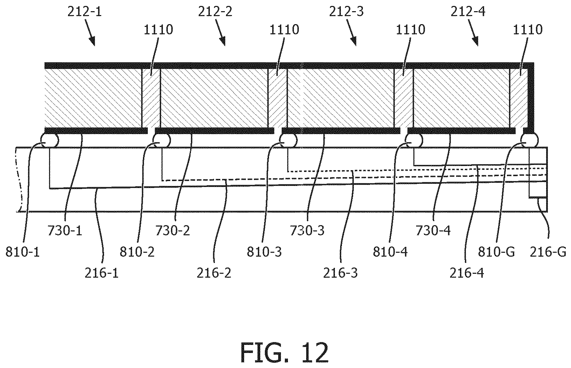

**Figure 12** shows a side, cross-sectional schematic view of another possible embodiment of the present disclosure. It may be desirable to reduce the number of ground interconnects in the system, by using filled kerfs with fewer ground returns. A variant of the above filled kerfs concept would not include numerous ground connections between elements. Instead, it could include fewer (or just one single) ground return connection. This connection could be made via electroplating, a conductive composite layer, soldering, conductive paste, etc. It could be located anywhere within the piezoelectric material (using the techniques listed elsewhere on this document) or could be at the edge, as illustrated. Composite materials could be added between elements for acoustic and structural purposes or a single, continuous block of piezoelectric could be used.

In the example shown in Figure 12, each transducer 212 includes a separately addressable bottom electrode 730, but is connected to the other transducers 212 via a shared ground electrode 720, made possible because the transducers 212 are separated by kerfs or channels filled with an insulating material 1110. In this example, bottom electrode 730-1 of transducer 212-1 is connected by stud bump or solder bump 810-1 to electrical trace 216-1, whereas bottom electrode 730-2 of transducer 212-2 is connected by stud bump or solder bump 810-2 to electrical trace 216-2, bottom electrode 730-3 of transducer 212-3 is connected by stud bump or solder bump 810-3 to electrical trace 216-3, and bottom electrode 730-4 of transducer 212-4 is connected by stud bump or solder bump 810-4 to electrical trace 216-4. Top electrode 720 covers all four transducers, and wraps continuously around a side of the piezoelectric block to connect with stud bump or solder bump 810-G, which connects to electrical trace 216-G.

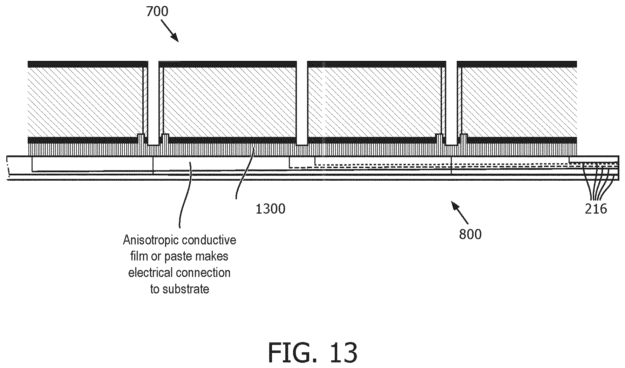

**Figure 13** shows a side, cross-sectional schematic view of another possible embodiment of the present disclosure. In other examples shown above, solder may be used to make an electrical connection between the transducer block 700 and the substrate 800. Alternatively, anisotropic conductive material 1300 (e.g., film or paste that conducts electricity only in a vertical direction while remaining insulative in a horizontal or diagonal direction) can be used to make this connection. This advantageously permits each electrical trace 216 to connect with an electrode immediately above it, without shorting to any other electrodes, which may simplify manufacturing.

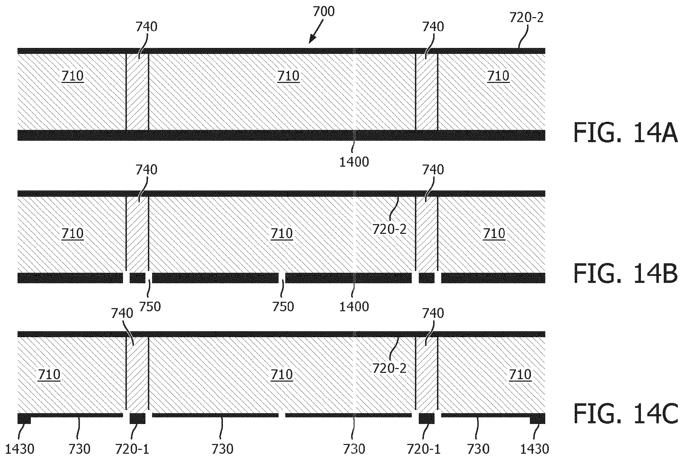

**Figures 14A** is a side, cross-sectional perspective view of another embodiment of the present disclosure that incorporates a conductive layer 1400 for electrical connection to the transducers. Yet another way to complete the electrical connection between the transducer block and the substrate is for one surface of the piezoelectric block (e.gt., the bottom surface) to include a conductive material, such as a deposited layer of metal or nickel graphite. This conductive material type and thickness may be selected for electrical, acoustic or mechanical purposes. In the example shown in Figure 14A, the piezoelectric block 700 is divided into subunits 710 by means of kerfs or channels filled with a conductive material 740. A second conductive material 1400 covers the lower surface of the piezoelectric block 700. The conductive material 1400 may be the same as, or different than, the conductive material 740.

**Figure 14B** is a side, cross-sectional perspective view of the embodiment of Figure 14A at a later stage of processing. In the example shown in Figure 14B, the conductive layer 1400 can be processed to create isolation kerfs 750, forming the bottom electrodes 720-1 and 730 as described above.

**Figure 14C** is a side, cross-sectional perspective view of the embodiment of Figure 14B at a later stage of processing. In the example shown in Figure 14C, portions of the electrodes 730 are thinned, leaving behind conductive posts 1430, whereas the electrodes 720-1 are left at their previous thickness, such that they may also serve as conductive ground posts. For some embodiments, this resulting structure aids manufacturing.

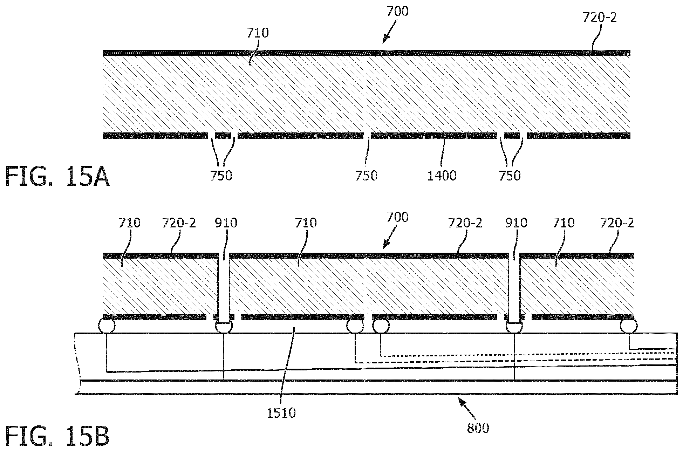

**Figure 15A** is a side, cross-sectional perspective view of another embodiment of the present disclosure wherein the piezoelectric block 700 does not include conductor-filled kerfs or channels. A conductive layer 1400 is deposited onto the bottom of the piezoelectric block 700, and then isolation kerfs 750 are cut.

**Figure 15B** is a side, cross-sectional perspective view of the embodiment of Figure 15A at a later stage of processing. The piezoelectric block 700 is joined to the flexible circuit assembly 800, with a backing material 1510. Trenches, channels, or kerfs 910 are then diced into the piezoelectric block 700, forming subunits 710. The backing material 1510 may be selected at least in part for its acoustic properties, to facilitate the operation of the transducer array.

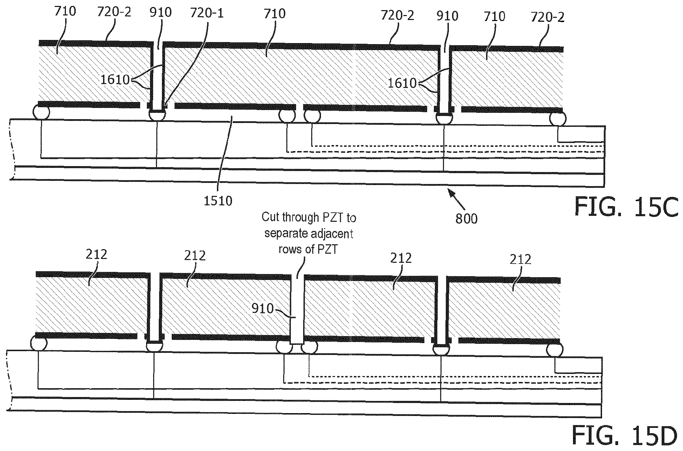

**Figure 15C** is a side, cross-sectional perspective view of the embodiment of Figure 15B at a later stage of processing. The diced trenches, channels, or kerfs 910 are coated with a conductive material 1610, which electrically connects the top ground electrodes 720-2 to the bottom ground electrodes 720-1

**Figure 15D** is a side, cross-sectional perspective view of the embodiment of Figure 15C at a later stage of processing. A trench, channel, or kerf 910 is then diced through the subunits 710, forming separate transducers 212.

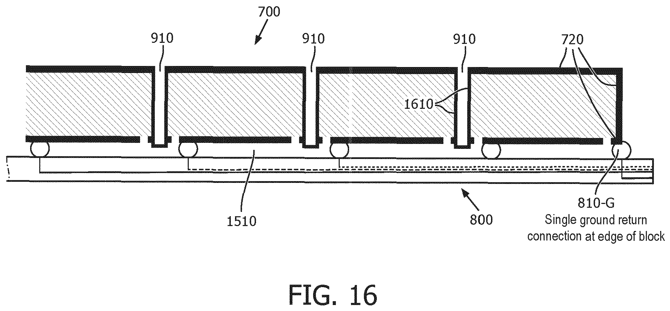

**Figure 16** is a side, cross-sectional perspective view of another embodiment of the present disclosure wherein trenches or kerfs 910 lined with conductor 1610 form a continuous top electrode 720 that wraps around a side of the piezoelectric block 700 to connect with a ground stud bump or solder bump 810G.

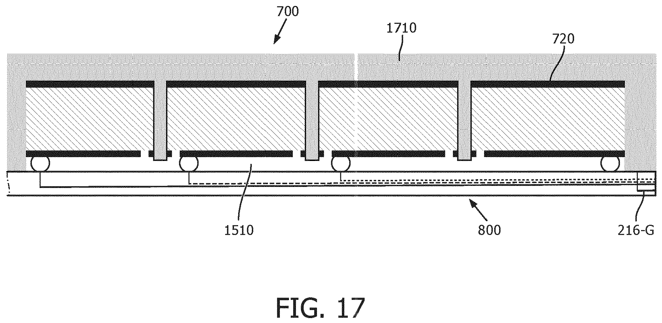

**Figure 17** is a side, cross-sectional perspective view of another embodiment of the present disclosure wherein a piezoelectric block 700, diced with trenches or kerfs 910, is filled and covered with a conductive material 1710 (e.g., a conductive epoxy, paste, solder, or potting compound), such that the conductive material 1710 electrically connects the top ground electrodes 720 to the ground trace 216-G. The conductive material 1710 may, like the backing material 1510, be selected at least in part for its acoustic properties, to facilitate the operation of the transducer array.

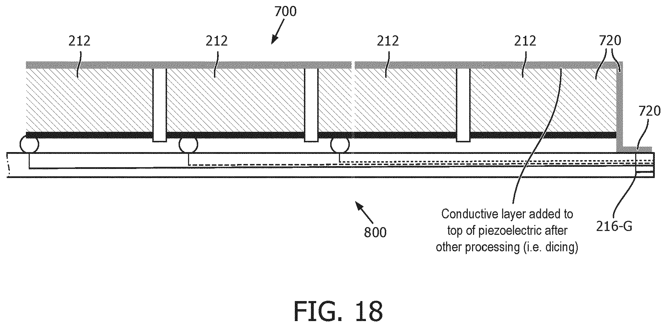

**Figure 18** is a side, cross-sectional perspective view of another embodiment of the present disclosure wherein the top electrode 720 comprises a conductive film or ribbon (e.g., gold foil or another thin, flexible conductive material) placed (e.g., adhered) across the tops of multiple transducers 212 and down a side of at least one transducer 212 to connect with a ground trace 216-G.

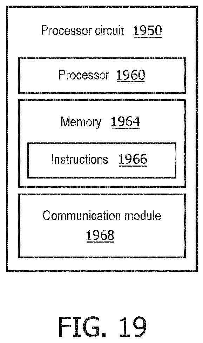

**Figure 19** is a schematic diagram of a processor circuit 1950, in accordance with aspects of the present disclosure. The processor circuit 1950 may be implemented in the ultrasound imaging system 100, or other devices or workstations (e.g., third-party workstations, network routers, etc.), or on a cloud processor or other remote processing unit, as necessary to implement the method. As shown, the processor circuit 1950 may include a processor 1960, a memory 1964, and a communication module 1968. These elements may be in direct or indirect communication with each other, for example via one or more buses.

The processor 1960 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1960 may also comprise another hardware device, a firmware device, or any combination thereof configured to perform the operations described herein. The processor 1960 may also be implemented as a combination of computing devices, e.g., a combination of a DSP and a microprocessor, a plurality of microprocessors, one or more microprocessors in conjunction with a DSP core, or any other such configuration.

The memory 1964 may include a cache memory (e.g., a cache memory of the processor 1960), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1964 includes a non-transitory computer-readable medium. The memory 1964 may store instructions 1966. The instructions 1966 may include instructions that, when executed by the processor 1960, cause the processor 1960 to perform the operations described herein. Instructions 1966 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1968 can include any electronic circuitry and/or logic circuitry to facilitate direct or indirect communication of data between the processor circuit 1950, and other processors or devices. In that regard, the communication module 1968 can be an input/output (I/O) device. In some instances, the communication module 1968 facilitates direct or indirect communication between various elements of the processor circuit 1950 and/or the ultrasound imaging system 100. The communication module 1968 may communicate within the processor circuit 1950 through numerous methods or protocols. Serial communication protocols may include but are not limited to US SPI, I²C, RS-232, RS-485, CAN, Ethernet, ARINC 429, MODBUS, MIL-STD-1553, or any other suitable method or protocol. Parallel protocols include but are not limited to ISA, ATA, SCSI, PCI, IEEE-488, IEEE-1284, and other suitable protocols. Where appropriate, serial and parallel communications may be bridged by a UART, USART, or other appropriate subsystem.

External communication (including but not limited to software updates, firmware updates, preset sharing between the processor and central server, or readings from the ultrasound device) may be accomplished using any suitable wireless or wired communication technology, such as a cable interface such as a USB, micro USB, Lightning, or FireWire interface, Bluetooth, Wi-Fi, ZigBee, Li-Fi, or cellular data connections such as 2G/GSM, 3G/UMTS, 4G/LTE/WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

Persons skilled in the art, after becoming familiar with the teachings herein, will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the appended claims.

## Claims

1. An intraluminal imaging device, comprising:
a flexible elongate member configured to be positioned within a body lumen of a patient, wherein the flexible elongate member comprises a longitudinal axis;
an ultrasound imaging assembly disposed at a distal portion of the flexible elongate member, wherein the ultrasound imaging assembly comprises:
a flexible substrate comprising a plurality of conductive traces, wherein the flexible substrate is disposed around a circumference of the flexible elongate member; and
a transducer array disposed on the flexible substrate such that the transducer array is disposed around the circumference of the flexible elongate member, wherein the transducer array comprises a plurality of rows and a plurality of columns, wherein each column comprises a plurality of transducer elements disposed along the longitudinal axis such that the plurality of transducer elements corresponds to the plurality of rows,
wherein the plurality of transducer elements comprises a first surface proximate to the flexible substrate and a second surface spaced from the flexible substrate, and
**characterized in that**
the plurality of transducer elements are electrically coupled to the plurality of conductive traces only on the first surface.

2. The device of claim 1, wherein the plurality of conductive traces comprises multiple conductive traces electrically coupled to the plurality of transducer elements, respectively, and configured to provide communication of electrical signals associated with ultrasound imaging,
wherein the first surface comprises a bottom surface and the second surface comprises a top surface, and
wherein the bottom surface comprises a bottom electrode electrically coupled to a respective conductive trace of the multiple conductive traces.

3. The device of claim 1, wherein the plurality of conductive traces comprises a single conductive trace at electrical ground and electrically coupled to each transducer element of plurality of transducer elements,
wherein the first surface comprises a bottom surface and the second surface comprises a top surface, and
wherein the top surface comprises a top electrode electrically coupled to the single conductive trace.

4. The device of claim 3, wherein the plurality of transducer elements comprises:
a side surface between the top surface and the bottom surface, wherein the side surface comprises a conductive material electrically coupled to the top electrode and the single conductive trace.

5. The device of claim 4, wherein the ultrasound imaging assembly further comprises a single electrical connection for the plurality of transducer elements providing the electrical ground via the single conductive trace.

6. The device of claim 4, wherein the bottom surface comprises:
a first bottom electrode portion in electrical communication with the side surface, the top electrode, and the single conductive trace; and
a second bottom electrode portion electrically isolated from the first bottom electrode portion by a discontinuity.

7. The device of claim 4,
wherein a first transducer element of the plurality of transducer elements and a second transducer element of the plurality of transducer elements are adjacent to one another,
wherein the conductive material on the side surface of the first transducer element is proximate to the conductive material on the side surface of the second transducer element, the device further comprising a single electrical connection electrically coupled to the conductive material on the side surface of the first transducer element and to the conductive material on the side surface of the second transducer element.

8. The device of claim 1, wherein the transducer array comprises gaps between the plurality of transducer elements.

9. The device of claim 1, wherein the ultrasound imaging assembly further comprises material disposed between side surfaces of the plurality of transducer elements such that no gaps are between the plurality of transducer elements,
wherein the plurality of conductive traces comprises a single conductive trace at electrical ground, and
wherein the material is conductive and electrically coupled to the single conductive trace such that the material provides the electrical ground to the plurality of transducer elements.

10. The device of claim 1, wherein the top surface comprises a top electrode that is continuous across the plurality of transducer elements.

11. The device of claim 1, wherein the ultrasound imaging assembly further comprises a plurality of solder bumps electrically coupling the plurality of transducer elements and the plurality of conductive traces.

12. The device of claim 1, wherein the ultrasound imaging assembly further comprises an anisotropic conductive material electrically coupling the plurality of transducer elements and the plurality of conductive traces.

13. The device of claim 1, wherein the ultrasound imaging assembly further comprises a conductive material layer electrically coupling the plurality of transducer elements and the plurality of conductive traces.

14. The device of claim 1,
further comprising a conductive layer disposed on the top surface,
wherein the plurality of conductive traces comprises a single conductive trace at electrical ground, and
wherein the conductive layer is electrically coupled to the single conductive trace such that the conductive layer provides the electrical ground to the plurality of transducer elements

15. The device of claim 1, wherein the device is an intravascular ultrasound (IVUS) imaging device, wherein the flexible elongate member is a catheter, wherein the body lumen is a vessel, wherein the ultrasound imaging assembly is an IVUS imaging assembly, and wherein the first surface is a bottom surface and the second surface is a top surface.

## Patentansprüche

1. Intraluminale Bildgebungsvorrichtung, umfassend:
ein flexibles längliches Element, das dafür konfiguriert ist, um innerhalb eines Körperlumens eines Patienten positioniert zu werden, wobei das flexible längliche Element eine Längsachse umfasst;
eine Ultraschall-Bildgebungsbaugruppe, die an einem distalen Abschnitt des flexiblen länglichen Elements angeordnet ist, wobei die Ultraschall-Bildgebungsbaugruppe Folgendes umfasst:
ein flexibles Substrat, das eine Vielzahl von Leiterbahnen umfasst, wobei das flexible Substrat um einen Umfang des flexiblen, länglichen Elements angeordnet ist; und
ein Wandlerarray, das auf dem flexiblen Substrat angeordnet ist, sodass das Wandlerarray um den Umfang des flexiblen länglichen Elements angeordnet ist, wobei das Wandlerarray eine Vielzahl von Zeilen und eine Vielzahl von Spalten umfasst, wobei jede Spalte eine Vielzahl von Wandlerelementen umfasst, die entlang der Längsachse angeordnet sind, sodass die Vielzahl von Wandlerelementen der Vielzahl von Zeilen entspricht,
wobei die Vielzahl von Wandlerelementen eine erste, dem flexiblen Substrat nahe liegende Oberfläche und eine zweite, vom flexiblen Substrat beabstandete Oberfläche umfasst, und
**dadurch gekennzeichnet, dass**
die Vielzahl von Wandlerelementen nur auf der ersten Oberfläche elektrisch mit der Vielzahl von Leiterbahnen verbunden ist.

2. Vorrichtung nach Anspruch 1, wobei die Vielzahl von Leiterbahnen mehrere Leiterbahnen umfasst, die jeweils elektrisch mit der Vielzahl von Wandlerelementen verbunden sind und zum Gewährleisten einer Übertragung elektrischer Signale, die mit Ultraschall-Bildgebung verknüpft sind, konfiguriert sind,
wobei die erste Oberfläche eine untere Oberfläche umfasst und die zweite Oberfläche eine obere Oberfläche umfasst und
wobei die untere Oberfläche eine untere Elektrode umfasst, die elektrisch mit einer jeweiligen Leiterbahn der mehreren Leiterbahnen verbunden ist.

3. Vorrichtung nach Anspruch 1, wobei die Vielzahl von Leiterbahnen eine einzige Leiterbahn an der elektrischen Masse und elektrisch mit jedem Wandlerelement der Vielzahl von Wandlerelementen verbunden umfasst,
wobei die erste Oberfläche eine untere Oberfläche umfasst und die zweite Oberfläche eine obere Oberfläche umfasst und
wobei die Oberseite eine obere Elektrode umfasst, die elektrisch mit der einzigen Leiterbahn verbunden ist.

4. Vorrichtung nach Anspruch 3, wobei die Vielzahl von Wandlerelementen Folgendes umfasst:
eine Seitenfläche zwischen der obere Oberfläche und der untere Oberfläche, wobei die Seitenfläche ein leitfähiges Material umfasst, das elektrisch mit der oberen Elektrode und der einzigen Leiterbahn verbunden ist.

5. Vorrichtung nach Anspruch 4, wobei die Ultraschall-Bildgebungsbaugruppe weiter eine einzige elektrische Verbindung für die Vielzahl von Wandlerelementen umfasst, welche die elektrische Masse über die einzige Leiterbahn bereitstellt.

6. Vorrichtung nach Anspruch 4, wobei die untere Oberfläche Folgendes umfasst:
einen ersten unteren Elektrodenabschnitt in elektrischer Verbindung mit der Seitenfläche, der oberen Elektrode und der einzigen Leiterbahn und
einen zweiten unteren Elektrodenabschnitt, der durch eine Unterbrechung elektrisch vom ersten unteren Elektrodenabschnitt isoliert ist.

7. Vorrichtung nach Anspruch 4,
wobei ein erstes Wandlerelement der Vielzahl von Wandlerelementen und ein zweites Wandlerelement der Vielzahl von Wandlerelementen zueinander benachbart sind,
wobei das leitfähige Material auf der Seitenfläche des ersten Wandlerelements nahe an dem leitfähigen Material auf der Seitenfläche des zweiten Wandlerelements liegt, wobei die Vorrichtung weiter eine einzige elektrische Verbindung umfasst, die elektrisch mit dem leitfähigen Material auf der Seitenfläche des ersten Wandlerelements und mit dem leitfähigen Material auf der Seitenfläche des zweiten Wandlerelements verbunden ist.

8. Vorrichtung nach Anspruch 1, wobei das Wandlerarray Lücken zwischen der Vielzahl von Wandlerelementen umfasst.

9. Vorrichtung nach Anspruch 1, wobei die Ultraschall-Bildgebungsbaugruppe weiter Material umfasst, das zwischen Seitenflächen der Vielzahl von Wandlerelementen angeordnet ist, sodass zwischen den Vielzahl von Wandlerelementen keine Lücken vorhanden sind,
wobei die Vielzahl von Leiterbahnen eine einzige Leiterbahn an der elektrischen Masse umfasst und
wobei das Material leitfähig und elektrisch mit der einzigen Leiterbahn verbunden ist, sodass das Material die elektrische Masse für die Vielzahl von Wandlerelementen bereitstellt.

10. Vorrichtung nach Anspruch 1, wobei die Oberseite eine obere Elektrode aufweist, die über die Vielzahl von Wandlerelementen durchgehend ist.

11. Vorrichtung nach Anspruch 1, wobei die Ultraschall-Bildgebungsbaugruppe weiter eine Vielzahl von Lötperlen umfasst, welche die Vielzahl von Wandlerelementen und die Vielzahl von Leiterbahnen elektrisch verbinden.

12. Vorrichtung nach Anspruch 1, wobei die Ultraschall-Bildgebungsbaugruppe weiter ein anisotropes leitfähiges Material umfasst, das die Vielzahl von Wandlerelementen und die Vielzahl von Leiterbahnen elektrisch verbindet.

13. Vorrichtung nach Anspruch 1, wobei die Ultraschall-Bildgebungsbaugruppe weiter eine leitfähige Materialschicht umfasst, die die Vielzahl von Wandlerelementen und die Vielzahl von Leiterbahnen elektrisch verbindet.

14. Vorrichtung nach Anspruch 1,
weiter umfassend eine leitfähige Schicht, die auf der Oberseite angeordnet ist,
wobei die Vielzahl von Leiterbahnen eine einzige Leiterbahn an der elektrischen Masse umfasst und
wobei die leitfähige Schicht elektrisch mit der einzigen Leiterbahn verbunden ist, sodass die leitfähige Schicht die elektrische Masse für die Vielzahl von Wandlerelementen bereitstellt.

15. Vorrichtung nach Anspruch 1, wobei die Vorrichtung eine intravaskuläre Ultraschall-Bildgebungsvorrichtung (IVUS) ist, wobei das flexible längliche Element ein Katheter ist, wobei das Körperlumen ein Gefäß ist, wobei die Ultraschall-Bildgebungsbaugruppe eine IVUS-Bildgebungsbaugruppe ist und wobei die erste Oberfläche eine untere Oberfläche ist und die zweite Oberfläche eine obere Oberfläche ist.

## Revendications

1. Dispositif d'imagerie intraluminale, comprenant :
un organe allongé flexible configuré pour être positionné dans une lumière corporelle d'un patient, dans lequel l'organe allongé flexible comprend un axe longitudinal ;
un ensemble d'imagerie ultrasonore disposé au niveau d'une partie distale de l'organe allongé flexible, dans lequel l'ensemble d'imagerie ultrasonore comprend :
un substrat flexible comprenant une pluralité de pistes conductrices, dans lequel le substrat flexible est disposé autour d'une circonférence de l'organe allongé flexible ; et
un réseau de transducteurs disposé sur le substrat flexible de telle sorte que le réseau de transducteurs soit disposé autour de la circonférence de l'organe allongé flexible, dans lequel le réseau de transducteurs comprend une pluralité de rangées et une pluralité de colonnes, dans lequel chaque colonne comprend une pluralité d'éléments transducteurs disposés le long de l'axe longitudinal de telle sorte que la pluralité d'éléments transducteurs corresponde à la pluralité de rangées,
dans lequel la pluralité d'éléments transducteurs comprend une première surface proche du substrat flexible et une seconde surface espacée du substrat flexible, et
**caractérisé en ce que**
la pluralité d'éléments transducteurs sont couplés électriquement à la pluralité de pistes conductrices uniquement sur la première surface.

2. Dispositif selon la revendication 1, dans lequel la pluralité de pistes conductrices comprend de multiples pistes conductrices couplées électriquement à la pluralité d'éléments transducteurs, respectivement, et configurées pour fournir une communication de signaux électriques associés à l'imagerie ultrasonore,
dans lequel la première surface comprend une surface inférieure et la seconde surface comprend une surface supérieure, et
dans lequel la surface inférieure comprend une électrode inférieure couplée électriquement à une piste conductrice respective des multiples pistes conductrices.

3. Dispositif selon la revendication 1, dans lequel la pluralité de pistes conductrices comprend une unique piste conductrice au niveau d'une masse électrique et couplée électriquement à chaque élément transducteur de la pluralité d'éléments transducteurs,
dans lequel la première surface comprend une surface inférieure et la seconde surface comprend une surface supérieure, et
dans lequel la surface supérieure comprend une électrode supérieure couplée électriquement à l'unique piste conductrice.

4. Dispositif selon la revendication 3, dans lequel la pluralité d'éléments transducteurs comprend :
une surface latérale entre la surface supérieure et la surface inférieure, dans lequel la surface latérale comprend un matériau conducteur couplé électriquement à l'électrode supérieure et à l'unique piste conductrice.

5. Dispositif selon la revendication 4, dans lequel l'ensemble d'imagerie ultrasonore comprend en outre une unique connexion électrique pour la pluralité d'éléments transducteurs fournissant la masse électrique via l'unique piste conductrice.

6. Dispositif selon la revendication 4, dans lequel la surface inférieure comprend :
une première partie d'électrode inférieure en communication électrique avec la surface latérale, l'électrode supérieure, et l'unique piste conductrice ; et
une seconde partie d'électrode inférieure isolée électriquement de la première partie d'électrode inférieure par une discontinuité.

7. Dispositif selon la revendication 4,
dans lequel un premier élément transducteur de la pluralité d'éléments transducteurs et un second élément transducteur de la pluralité d'éléments transducteurs sont adjacents l'un à l'autre,
dans lequel le matériau conducteur sur la surface latérale du premier élément transducteur est proche du matériau conducteur sur la surface latérale du second élément transducteur, le dispositif comprenant en outre une unique connexion électrique couplée électriquement au matériau conducteur sur la surface latérale du premier élément transducteur et au matériau conducteur sur la surface latérale du second élément transducteur.

8. Dispositif selon la revendication 1, dans lequel le réseau de transducteurs comprend des espaces entre la pluralité d'éléments transducteurs.

9. Dispositif selon la revendication 1, dans lequel l'ensemble d'imagerie ultrasonore comprend en outre un matériau disposé entre des surfaces latérales de la pluralité d'éléments transducteurs de telle sorte qu'il n'y ait pas d'espace entre la pluralité d'éléments transducteurs,
dans lequel la pluralité de pistes conductrices comprend une unique piste conductrice au niveau d'une masse électrique, et
dans lequel le matériau est conducteur et couplé électriquement à l'unique piste conductrice de telle sorte que le matériau fournisse la masse électrique à la pluralité d'éléments transducteurs.

10. Dispositif selon la revendication 1, dans lequel la surface supérieure comprend une électrode supérieure qui est continue parmi la pluralité d'éléments transducteurs.

11. Dispositif selon la revendication 1, dans lequel l'ensemble d'imagerie ultrasonore comprend en outre une pluralité de perles de soudure couplant électriquement la pluralité d'éléments transducteurs et la pluralité de pistes conductrices.

12. Dispositif selon la revendication 1, dans lequel l'ensemble d'imagerie ultrasonore comprend en outre un matériau conducteur anisotrope couplant électriquement la pluralité d'éléments transducteurs et la pluralité de pistes conductrices.

13. Dispositif selon la revendication 1, dans lequel l'ensemble d'imagerie ultrasonore comprend en outre une couche de matériau conducteur couplant électriquement la pluralité d'éléments transducteurs et la pluralité de pistes conductrices.

14. Dispositif selon la revendication 1,
comprenant en outre une couche conductrice disposée sur la surface supérieure,
dans lequel la pluralité de pistes conductrices comprend une unique piste conductrice au niveau d'une masse électrique, et
dans lequel la couche conductrice est couplée électriquement à l'unique piste conductrice de telle sorte que la couche conductrice fournisse la masse électrique à la pluralité d'éléments transducteurs.

15. Dispositif selon la revendication 1, dans lequel le dispositif est un dispositif d'imagerie ultrasonore intravasculaire (IVUS), dans lequel l'organe allongé flexible est un cathéter, dans lequel la lumière corporelle est un vaisseau, dans lequel l'ensemble d'imagerie ultrasonore est un ensemble d'imagerie IVUS, et dans lequel la première surface est une surface inférieure et la seconde surface est une surface supérieure.
